# EUROPEAN PATENT SPECIFICATION

(11) **EP 2 430 930 B1**
(45) Date of publication and mention of the grant of the patent: **04.10.2017**
(21) Application number: 10775066.3
(22) Date of filing: 04.05.2010
(51) Int. Cl.: A41D 1/06, A41D 13/05, A41D 27/00, A41D 1/12, A41D 13/00, A61F 5/01

(54) **BODY-CORRECTING TROUSERS**
KÖRPERKORRIGIERENDE HOSE
PANTALON POUR LA CORRECTION DE LA SILHOUETTE

(30) Priority: 15.05.2009 KR 20090042728
(43) Date of publication of application: 21.03.2012
(73) Proprietor: Cho, Jong Dae, Suwon-si, Gyeonggi-Do 441-460 (KR)
(72) Inventor: Cho, Jong Dae, Suwon-si, Gyeonggi-Do 441-460 (KR)
(74) Representative: Schumacher & Willsau
(86) International application number: PCT/KR2010/002831
(87) International publication number: WO 2010/131864

(56) References cited:
- JP-A- 10 280 209
- JP-A- 11 061 516
- JP-A- 2007 023 466
- KR-B1- 100 527 561

## Description

### [Technical Field]

The present invention relates to body shape-correcting trousers, and more particularly, to body shape-correcting trousers in which parts of the trousers have different densities to generate forces for externally rotating a wearer's legs around the pelvis and to thus correct bowed legs or "O"-shaped legs.

### [Background Art]

Normal legs have a desirable angle between the femur and tibia and also have a balanced distance between left and right knee joints. As most of people have their hip and knee joints dislocated, however, they have bowed legs like "O"-shaped legs.

The formation of the bowed legs is caused by heredity, racial differences and diseases, but generally, the causes of the generation of the bowed legs have been not completely revealed yet. However, the bowed legs are generated by more acquired causes than congenial causes, and the most common examples of the acquired causes are lifestyle habits and postures such as carrying a baby on the back, lack of exercise, ageing, wrong walking activities and so on. Especially, since typical floor-sitting cultures in oriental people cause the internal rotation deformity of the hip joint near the pelvis, the bowed legs are commonly found in more the oriental people than the westerners.

If the bowed legs, especially, the "O"-shaped legs are formed, they are not good in the cosmetic appearance, and further, they are unstable in the standing or walking posture. Also, the outside distance between the knee joints becomes open, and the inside distance therebetween becomes close, such that the cartilage positioned inside the knee to perform a lubrication action becomes high in the frictional pressure thereof, which easily causes degenerative arthritis.

Therefore, much focus is given to the correction of the "O"-shaped legs so as to improve their outer appearance and health, and thus, a variety of methods for correcting them have been introduced. As typical methods, first, there is surgical correction for the bowed legs, and alternatively, the correction for the bowed legs is performed by using a brace or corrector.

However, the surgical correction or the use of the brace or corrector may cause adverse effects, the possibility of mental stress according to the surgery, and high costs. As a relatively simple method, the bowed legs are tied by belts, but in this case, just the knees are pressed toward the insides thereof, which gives no influence on the correction of the internal rotation deformity of the hip joint.

On the other hand, shoe soles or socks are used to simply correct the axis of the weight of the lower body, which gives a little good influence on the correction of the bowed legs, but they provide such effects only when they are worn. That is, the shoe soles or socks do not have any function of correcting the bowed legs, but they have a function of adjusting the bowed legs just while worn. So as to obtain desirable effects for the bowed leg correction, the three-dimensional alignment of the human body should be corrected, and therefore, such one-dimensional or two-dimensional belts, shoe soles and socks just provide slight correction effects.

So as to correct the internal rotation of the femur and to thus correct the bowed legs, there is disclosed Korean Patent Laid-open Application No. 2000-0053733 entitled "body shape-correcting method and implement".

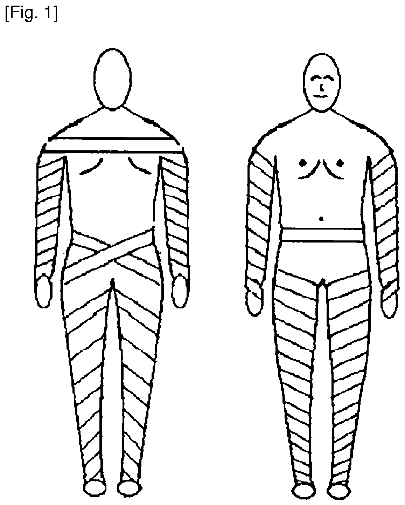

FIG.1 shows a conventional body shape-correcting implement, and as shown in FIG.1, a body shape-correcting implement is configured to spirally wind a wide elastic band around arms or legs, such that while the wearer is lying in bed, a rotary force is applied to him or her, thereby correcting his or her body shape.

According to the conventional body shape-correcting implement, however, the elastic band should be wound every correction, and further, the correction is achieved only by performing the exercise in the bed manufactured specifically, such that it is very inconvenient for people to use in their daily life. Another document EP 1 342 432 A1 discloses a garment having functions of assisting the stability of the hip joint and reducing lumbar lordosis.

### [Technical Problem]

Accordingly, the present invention has been made in view of the above-mentioned problems occurring in the prior art, and it is an object of the present invention to provide body shape-correcting trousers in which parts of a trousers fabric are woven to have different densities to generate forces for externally rotating a wearer's femur and tibia and to thus allow the wearer's pelvis inclined forwardly to be erected, such that while he or she is wearing the trousers in his or her daily life, his or her "O"-shaped legs or inclined pelvis can be smoothly corrected.

It is another object of the present invention to provide body shape-correcting trousers that are capable of continuously performing the external rotation for muscles, such that even when the trousers are not worn, the body shape-correcting effect can be still maintained.

### [Technical Solution]

To accomplish the above objects, according to the present invention, there are provided body shape-correcting trousers having parts of a trousers fabric woven to have different densities so as to generate forces for externally rotating a wearer's leg joints, wherein a thigh-rotating band is spirally turned in an external rotation direction of the thigh in such a manner as to be extended downwardly from the lower abdomen to the upper end of the knee cap, the thigh-rotating band being woven to have a relatively higher density than other parts of the trousers.

### [Advantageous Effect]

According to the present invention, the body shape-correcting trousers enable the O-shaped legs of the wearer to be simply corrected in everyday life, enable his or her inclined pelvis to be just erected, and enable his or her leg joints to be kept in a correct posture, thereby preventing arthritis or aging cartilage.

In addition, the body shape-correcting trousers enable the legs of the wearer to be stretched straightly, thereby providing good visual appearance.

### [Description of Drawings]

FIG.1 is an exemplary view showing a conventional body shape-correcting implement.
FIG.2 is a dissected view showing a structure of thigh muscles.
FIG.3 is a front view showing a structure of body shape-correcting trousers according to the present invention.
FIG.4 is a rear view showing the structure of the body shape-correcting trousers of FIG.3.
FIG.5 is a front view showing the rotating directions on the body shape-correcting trousers according to the present invention.
FIG.6 is a rear view showing the rotating directions on the body shape-correcting trousers according to the present invention.
FIG.7 is a side view showing external rotation exercise directions during a wearer's walking.

### [Best Mode for Invention]

According to the present invention, there are provided body shape-correcting trousers having parts of a trousers fabric woven to have different densities so as to generate forces for externally rotating a wearer's leg joints, wherein a thigh-rotating band is spirally turned in an external rotation direction of the thigh in such a manner as to be extended downwardly from the lower abdomen to the upper end of the knee cap, the thigh-rotating band being woven to have a relatively higher density than other parts of the trousers.

Further, the body shape-correcting trousers have a calf-rotating band spirally turned in an external rotation direction of the calf in such a manner as to be extended downwardly from the lower end of the knee cap to the middle of the calf, the calf-rotating band being woven to have a relatively higher density than the thigh-rotating band.

Further, the body shape-correcting trousers have a gluteus maximus muscle-supporting band adapted to support the lower end portion of the gluteus maximus muscle in such a manner as to be extended downwardly and backwardly from the lower abdomen to left and right sides and to be connected to each other on the lower side of the hip and having a front gluteus maximus muscle-supporting band positioned on the lower abdomen and a rear gluteus maximus muscle-supporting band positioned on the lower side of the hip, the rear gluteus maximus muscle-supporting band being woven to have a relatively higher density than the front gluteus maximus muscle-supporting band.

In addition, the body shape-correcting trousers have a gluteus medius muscle-rotating band adapted to surround the gluteus medius muscle in such a manner as to be extended forwardly from the upper side of the hip to left and right sides and to be connected at the ends to one sides of the front side thigh-rotating band and having a front gluteus medius muscle-rotating band positioned on the front side of a human body and a rear gluteus medius muscle-rotating band positioned on the upper side of the hip, the rear gluteus medius muscle-rotating band being woven to have a relatively higher density than the front gluteus medius muscle-rotating band.

According to the present invention, further, the body shape-correcting trousers have a knee-supporting band adapted to surround the neighboring region of the knee cap to a shape selected from rhombus, hexagon and circle, the knee-supporting band being woven to have a relatively higher density than the thigh-rotating band and to have a relatively lower density than the calf-rotating band.

Furthermore, the body shape-correcting trousers have a rear knee-supporting band spirally turned in an external rotation direction from the upper end of the knee-supporting band in such a manner as to be connected to the lower end of the knee-supporting band, the rear knee-supporting band being woven to have the same density as the knee-supporting band.

### [Mode for Invention]

Hereinafter, an explanation on body shape-correcting trousers according to a preferred embodiment of the present invention will be in detail given with reference to the attached drawings.

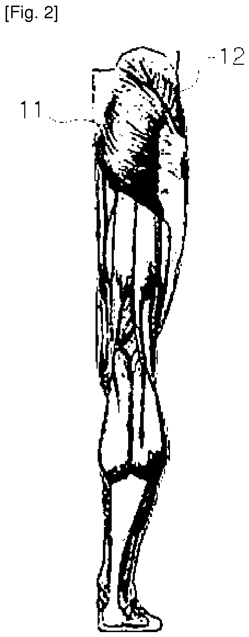

FIG.2 is a dissected view showing a structure of thigh muscles.

A gluteus maximus muscle 11, which externally rotates the femur, is located on the upper side of the thigh in such a manner as to be extended slantly downwardly from the center of the coccyx and to be thus connected to the left and right sides. Further, a gluteus medius muscle 12 is located on the upper side of the gluteus maximus muscle 11 in such a manner as to be extended slantly downwardly.

The gluteus maximus muscle 11 is one of the gluteal muscles positioned on the back surface of the pelvis, which is the largest in the gluteal muscles, located on the topmost layer of the hip, and divided into several bundles through connective tissues positioned at the inside thereof.

The gluteus maximus muscle 11 serves to pull the legs backwardly, to fix the legs, and to pull the pelvis and the trunk backwardly to allow the human body to be erected. Therefore, the gluteus maximus muscle 11 is very important to take an upright posture. Further, the gluteus maximus muscle 11 as an antagonist of the Iliopsoas is the extensor of the hip joint, and the Iliopsoas is the flexor of the hip joint, such that the gluteus maximus muscle 11 and the Iliopsoas are in turn used when the legs move up and down.

The gluteus maximus muscle 11 and the gluteus medius muscle 12 are the external rotation muscles of the femur, and if a force is applied to the gluteus maximus muscle 11 and the gluteus medius muscle 12 to perform the external rotation, the femur and the tibia are externally rotated to correct the O-shaped legs.

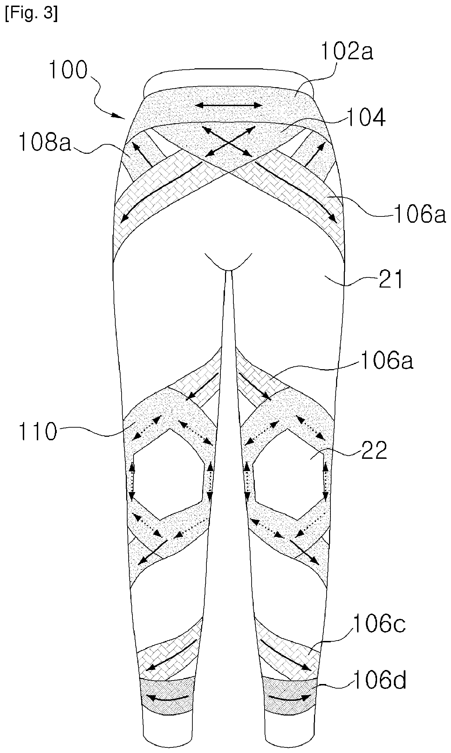

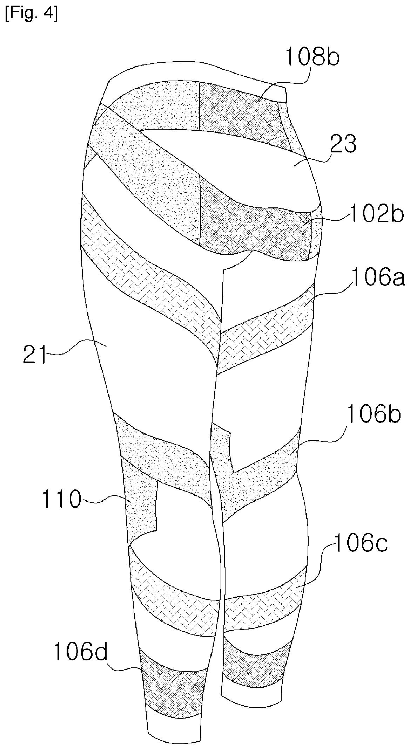

FIG.3 is a front view showing a structure of body shape-correcting trousers according to the present invention, and FIG.4 is a rear view showing the structure of the body shape-correcting trousers of FIG.3.

The body shape-correcting trousers 100 of the present invention are configured wherein a fabric is woven in different densities to generate a rotating force caused by the pressure differences thereof. If the band-shaped portions of the trousers 100 are woven more tensely, they have larger pressures than other portions to generate pulling forces along the directions of the lengths of the bands. When the trousers 100 are worn, the pulling forces are changed to forces for rotating the leg joints, with which the muscles and bones are pulled to given directions.

The fabric of the trousers 100 has different densities, while is being woven as a unitary body, and therefore, the trousers 100 and the bands normally generate the rotating force therefrom, without any separation therebetween. Moreover, if the fabric formed to a unitary body has different densities, the frictional force against the skin surface becomes increased to enable the force for rotating the muscles to a desired direction to be strengthened.

A thigh region 21, a knee cap region 22, and a gluteus maximus muscle region 23, where no band is formed, are woven with a general fabric to thus have the lowest density in all of the regions of the trousers 100.

A front gluteus maximus muscle-supporting band 102a is located on the front lower abdomen of the body shape-correcting trousers 100.

The front gluteus maximus muscle-supporting band 102a is extended downwardly and backwardly from the lower abdomen to left and right sides, and the both ends of the extended band to the left and right sides are connected to each other on the lower side of the hip to form a rear gluteus maximus muscle-supporting band 102b. The gluteus maximus muscle is positioned just on the upper side of the hip, and thus, the rear gluteus maximus muscle-supporting band 102b serves to support the lower end of the gluteus maximus muscle. The front gluteus maximus muscle-supporting band 102a is positioned on the front side of the trousers 100, and the rear gluteus maximus muscle-supporting band 102b is on the lower side of the hip, so that the lower side of the hip can be lifted slantly toward the lower abdomen.

Also, the rear gluteus maximus muscle-supporting band 102b is woven to have a relatively higher density than the front gluteus maximus muscle-supporting band 102a, such that a pulling force in a direction from the front gluteus maximus muscle-supporting band 102a toward the rear gluteus maximus muscle-supporting band 102b is generated to enable the topmost region of the thigh to be externally rotated.

A connection portion 104 is located just on the lower side of the middle portion of the front gluteus maximus muscle-supporting band 102a, and a thigh-rotating band 106a is extended downwardly from the connection portion 104.

The thigh-rotating band 106a is spirally turned in an external rotation direction of the thigh in such a manner as to be extended downwardly from the lower abdomen on which the connection portion 104 is located to the upper end of the knee cap. That is, the thigh-rotating band 106a is spirally turned downwardly in a clockwise direction along the right leg and in a counterclockwise direction along the left leg, such that the right leg is externally rotated in the clockwise direction and the left leg in the counterclockwise direction.

The thigh-rotating band 106a is turned downwardly one revolution up to the upper end of the knee cap, but if necessary, it may be turned two or three revolutions.

The thigh-rotating band 106a is woven to have a relatively higher density than the regions where no band is formed, but the thigh-rotating band 106a has a relatively lower density than the front and rear gluteus maximus muscle-supporting bands 102a and 102b or the connection portion 104.

In addition, the body shape-correcting trousers 100 have a gluteus medius muscle-rotating band 108 connected to the upper sides of the thigh-rotating band 106a. The gluteus medius muscle-rotating band 108 is divided into a front gluteus medius muscle-rotating band 108a and a rear gluteus medius muscle-rotating band 108b. The front gluteus medius muscle-rotating band 108a is located on portions to which the thigh-rotating band 106a are connected, and the rear gluteus medius muscle-rotating band 108b is located to surround the gluteus medius muscle of the back of the waist.

The rear gluteus medius muscle-rotating band 108b is woven to have a relatively higher density than the front gluteus medius muscle-rotating band 108a, thereby generating an external rotating force in a direction from the front gluteus medius muscle-rotating band 108a to the rear gluteus medius muscle-rotating band 108b.

The rear gluteus medius muscle-rotating band 108b is woven to have a similar density to the rear gluteus maximus muscle-supporting band 102b.

Further, the body shape-correcting trousers have a knee-supporting band 110 located around the knee cap. The knee-supporting band 110 is woven to a shape selected from rhombus, hexagon and circle and serves to surround the neighboring region of the knee cap to generate a force for opening the knee in every direction.

The knee-supporting band 110 is woven to have a relatively higher density than the thigh-rotating band 106a and to have a relatively lower density than the rear gluteus maximus muscle-supporting band 102b or the rear gluteus medius muscle-rotating band 108b.

Furthermore, the knee-supporting band 110 is connected on the upper end thereof to the end of the thigh-rotating band 106a, and in this case, the density of the knee-supporting band 110 is higher than that of the thigh-rotating band 110, thereby generating a force for pulling in a direction from the thigh-rotating band 106a to the knee-supporting band 110.

In addition, the body shape-correcting trousers have a rear knee-supporting band 106b spirally turned around the rear of the knee in such a manner as to be extended downwardly from the upper end of the knee-supporting band 110 to the lower end of the knee-supporting band 110. In the same manner as the thigh-rotating band 106a, the rear knee-supporting band 106b is turned downwardly in the external rotation direction (in the clockwise direction along the right leg and in the counterclockwise direction along the left leg).

The rear knee-supporting band 106b serves to send the pulling force in a direction from the thigh-rotating band 106a toward the knee-supporting band 110 to a calf-rotating band 106c, without stopping. The rear knee-supporting band 106b is woven to have the same density as the knee-supporting band 110.

The calf-rotating band 106c is spirally turned in an external rotation direction of the calf in such a manner as to be extended downwardly from the lower end of the knee cap where the lower end of the knee-supporting band 110 is located just to the upper side of the ankle (the lower end of the gastrocnemius). The calf-rotating band 106c is turned in the same direction as the thigh-rotating band 106 and is woven to have a relatively higher density than the knee-supporting band 110.

Further, an ankle-supporting band 106d is connected to the lower side of the calf-rotating band 106 and serves to pull the calf-rotating band 106.

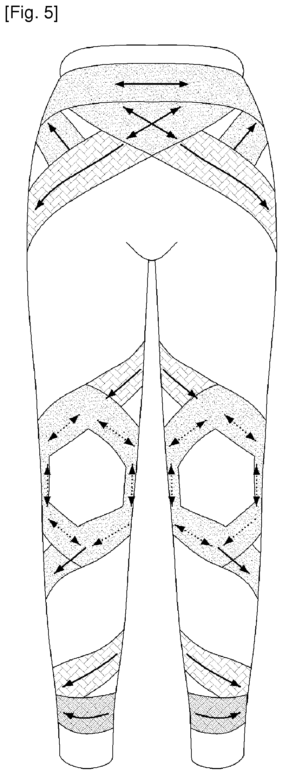

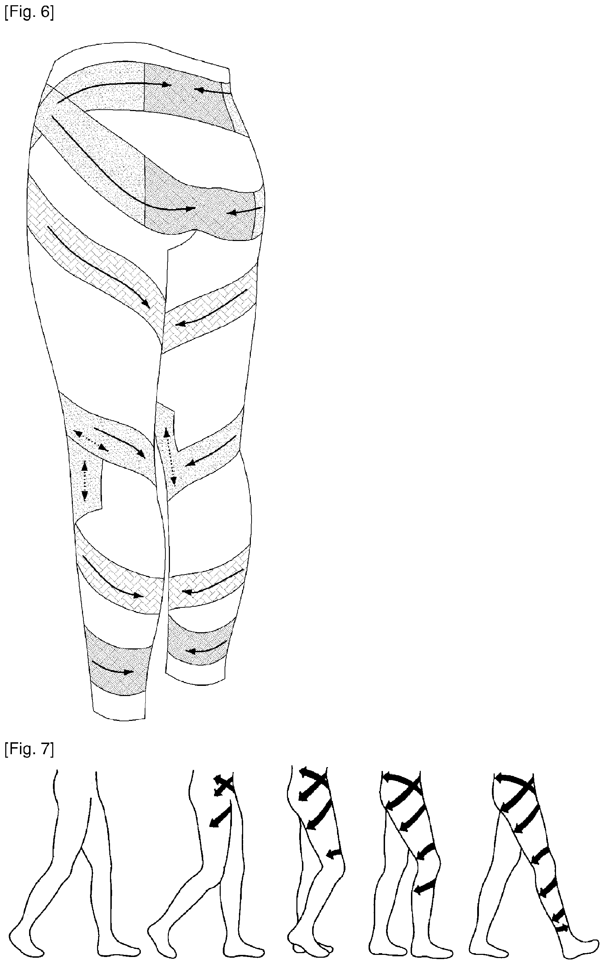

On the other hand, FIG.5 is a front view showing the rotating directions on the body shape-correcting trousers according to the present invention, and FIG.6 is a rear view showing the rotating directions on the body shape-correcting trousers according to the present invention.

As shown in FIGS.5 and 6, the externally rotating forces in the clockwise and counterclockwise directions are generated on left and right thighs 21, such that the left and right thighs 21 are rotated around the hip joint, thereby correcting the bowed legs.

Further, the extending forces along the outside boundary lines around knee cap 22 are generated to distribute the load applied to the knee.

FIG.7 is a side view showing external rotation exercise directions occurring while a wearer wearing the body shape-correcting trousers 100 is walking.

As shown in the second picture from the left of FIG.7, first, the wearer lifts his leg forward so as to walk (which is knee flexion exercise), such that the external rotation exercise is generated on the hip region (for example, the gluteus maximus muscle and the gluteus medius muscle) by the formation of the thigh-rotating band. The external rotation exercise of the leg caused by the knee flexion exercise during walking is changed to the knee extension exercise, thereby sending the external rotation exercise to the hip, thigh, knee, calf and ankle, serially. Therefore, while the wearer is walking, his or her legs are in turn moved to perform the external rotation exercises.

Even though not shown in FIG.7, the rear leg (that is, the left leg) of the wearer performs the external rotation exercise, in the same manner as the front leg (that is, the right leg), and as the wearer moves forwardly, the external rotation occurs on the left and right legs in turn.

Further, if the wearer wearing the body shape-correcting trousers 100 walks, his or her hip joint is kept rotated externally, such that his or her pelvis inclined forwardly can be erected straightly. Moreover, the external rotation muscles (for example, the gluteus maximus muscle, the gluteus medius muscle, piriformis muscle, superior gemellus muscle, obturator internus muscle, inferior gemellus muscle and obturator externus muscle) are strengthened to develop the tendons and muscles in a balanced manner. Therefore, the alignment from the pelvis to the legs including the thighs, knees, and calves is erectly made to provide the correct walking posture and the correction of the bowed legs.

While the present invention has been described with reference to the particular illustrative embodiments, it is not to be restricted by the embodiments but only by the appended claims. It is to be appreciated that those skilled in the art can change or modify the embodiments without departing from the scope of the present invention.

## Claims

1. Body shape-correcting trousers having parts of a trousers fabric woven to have different densities so as to generate forces for externally rotating a wearer's leg joints, the body shape-correcting trousers comprising:
a thigh-rotating band 106a spirally turned in an external rotation direction of the thigh in such a manner as to be extended downwardly from the lower abdomen to the upper end of the knee cap; and
a calf-rotating band 106c spirally turned in an external rotation direction of the calf in such a manner as to be extended downwardly from the lower end of the knee cap to the middle of the calf,
wherein the thigh-rotating band 106a is woven to have a relatively higher density than other parts of the trousers and the calf-rotating band 106c is woven to have a relatively higher density than the thigh-rotating band 106a.

2. Body shape-correcting trousers having parts of a trousers fabric woven to have different densities so as to generate forces for externally rotating a wearer's leg joints, the body shape-correcting trousers comprising:
a thigh-rotating band 106a spirally turned in an external rotation direction of the thigh in such a manner as to be extended downwardly from the lower abdomen to the upper end of the knee cap; and
a gluteus maximus muscle-supporting band 102 adapted to support the lower end portion of the gluteus maximus muscle in such a manner as to be extended downwardly and backwardly from the lower abdomen to left and right sides and to be connected to each other on the lower side of the hip,
wherein the thigh-rotating band 106a is woven to have a relatively higher density than other parts of the trousers and a rear gluteus maximus muscle-supporting band 102b positioned on the lower side of the hip in the gluteus maximus muscle-supporting band 102 is woven to have a relatively higher density than a front gluteus maximus muscle-supporting band 102a positioned on the lower abdomen in the gluteus maximus muscle-supporting band 102.

3. The body shape-correcting trousers according to claim 2, further comprising a gluteus medius muscle-rotating band 108 adapted to surround the gluteus medius muscle in such a manner as to be extended forwardly from the upper side of the hip to left and right sides and to be connected at the ends to one sides of the front side thigh-rotating band 106a, a rear gluteus medius muscle-rotating band 108b positioned on the upper side of the hip in the gluteus medius muscle-rotating band 108 being woven to have a relatively higher density than a front gluteus medius muscle-rotating band 108a positioned on the front side of a human body in the gluteus medius muscle-rotating band 108.

4. The body shape-correcting trousers according to claim 1, further comprising a knee-supporting band 110 adapted to surround the neighboring region of the knee cap to a shape selected from rhombus, hexagon and circle, the knee-supporting band 110 being woven to have a relatively higher density than the thigh-rotating band 106a and to have a relatively lower density than the calf-rotating band 106c.

5. The body shape-correcting trousers according to claim 4, further comprising a rear knee-supporting band 106b spirally turned in an external rotation direction from the upper end of the knee-supporting band 110 in such a manner as to be connected to the lower end of the knee-supporting band 110, the rear knee-supporting band 106b being woven to have the same density as the knee-supporting band 110.

## Patentansprüche

1. Körperformkorrigierende Hose mit Teilen eines Hosengewebes, die so gewebt sind, dass sie unterschiedliche Dichten aufweisen, um Kräfte zur Außenrotation der Beingelenke einer Trägerperson zu erzeugen, wobei die körperformkorrigierende Hose umfasst:
ein Oberschenkeldrehband 106a, das in einer Außenrotationsrichtung des Oberschenkels in einer solchen Weise spiralförmig gedreht ist, dass es ausgehend vom Unterbauch nach unten bis zum oberen Ende der Kniescheibe gestreckt ist; und
ein Wadendrehband 106c, das in einer Außenrotationsrichtung der Wade in einer solchen Weise spiralförmig gedreht ist, dass es ausgehend vom unteren Ende der Kniescheibe nach unten bis zur Mitte der Wade gestreckt ist,
wobei das Oberschenkeldrehband 106a so gewebt ist, dass es eine relativ höhere Dichte als andere Teile der Hose aufweist, und das Wadendrehband 106c so gewebt ist, dass es eine relativ höhere Dichte als das Oberschenkeldrehband 106a aufweist.

2. Körperformkorrigierende Hose mit Teilen eines Hosengewebes, die so gewebt sind, dass sie unterschiedliche Dichten aufweisen, um Kräfte zur Außenrotation der Beingelenke einer Trägerperson zu erzeugen, wobei die körperformkorrigierende Hose umfasst:
ein Oberschenkeldrehband 106a, das in einer Außenrotationsrichtung des Oberschenkels in einer solchen Weise spiralförmig gedreht ist, dass es ausgehend vom Unterbauch nach unten bis zum oberen Ende der Kniescheibe gestreckt ist; und
ein Glutaeus-Maximus-Muskelstützband 102, das eingerichtet ist, um den unteren Endabschnitt des großen Gesäßmuskels Glutaeus Maximus in einer solchen Weise zu stützen, dass es ausgehend vom Unterbauch nach unten und nach hinten zur linken und rechten Seite gestreckt ist und an der unteren Seite der Hüfte miteinander verbunden ist,
wobei das Oberschenkeldrehband 106a so gewebt ist, dass es eine relativ höhere Dichte als andere Teile der Hose aufweist, und ein hinteres Glutaeus-Maximus-Muskelstützband 102b, das an der unteren Seite der Hüfte im Glutaeus-Maximus-Muskelstützband 102 positioniert ist, so gewebt ist, dass es eine relativ höhere Dichte aufweist als ein vorderes Glutaeus-Maximus-Muskelstützband 102a, das am Unterbauch im Glutaeus-Maximus-Muskelstützband 102 positioniert ist.

3. Körperformkorrigierende Hose nach Anspruch 2, ferner umfassend: ein Glutaeus-Medius-Muskeldrehband 108, das eingerichtet ist, um den mittigen Gesäßmuskel Glutaeus Medius in einer solchen Weise zu umgeben, dass es ausgehend von der oberen Seite der Hüfte nach vorn zur linken und rechten Seite gestreckt ist und an den Enden mit einer Seite des vorderseitigen Oberschenkeldrehbands 106a verbunden ist, wobei ein hinteres Glutaeus-Medius-Muskeldrehband 108b, das an der oberen Seite der Hüfte im Glutaeus-Medius-Muskeldrehband 108 positioniert ist, so gewebt ist, dass es eine relativ höhere Dichte aufweist als ein vorderes Glutaeus-Medius-Muskeldrehband 108a, das an der Vorderseite eines menschlichen Körpers im Glutaeus-Medius-Muskeldrehband 108 positioniert ist.

4. Körperformkorrigierende Hose nach Anspruch 1, ferner umfassend: ein Kniestützband 110, das eingerichtet ist, um den Nachbarbereich der Kniescheibe in einer Form zu umgeben, die gewählt ist aus Rhombus, Sechseck und Kreis, wobei das Kniestützband 110 so gewebt ist, dass es eine relativ höhere Dichte als das Oberschenkeldrehband 106a aufweist und eine relativ niedrigere Dichte als das Wadendrehband 106c aufweist.

5. Körperformkorrigierende Hose nach Anspruch 4, ferner umfassend: ein hinteres Kniestützband 106b, das in einer Außenrotationsrichtung ausgehend vom oberen Ende des Kniestützbands 110 in einer solchen Weise spiralförmig gedreht ist, dass es mit dem unteren Ende des Kniestützbands 110 verbunden ist, wobei das hintere Kniestützband 106b so gewebt ist, dass es die gleiche Dichte wie das Kniestützband 110 aufweist.

## Revendications

1. Pantalon de correction de silhouette comportant des parties d'une étoffe de pantalon tissées de façon à présenter différentes densités afin de générer des forces pour faire tourner extérieurement les articulations des jambes d'une personne le portant, le pantalon de correction de silhouette comprenant :
une bande de rotation de cuisse 106a enroulée en spirale dans une direction de rotation extérieure de la cuisse de telle sorte qu'elle s'étende vers le bas du bas-ventre à l'extrémité supérieure de la patella ; et
une bande de rotation de mollet 106c enroulée dans une direction de rotation extérieure du mollet de telle sorte qu'elle s'étende vers le bas de l'extrémité inférieure de la patella à la partie médiane du mollet,
la bande de rotation de cuisse 106a étant tissée de façon à présenter une densité relativement plus élevée que d'autres parties du pantalon et la bande de rotation de mollet 106c étant tissée de façon à présenter une densité relativement plus élevée que la bande de rotation de cuisse 106a.

2. Pantalon de correction de silhouette comportant des parties d'une étoffe de pantalon tissées de façon à présenter différentes densités afin de générer des forces pour faire tourner extérieurement les articulations des jambes d'une personne le portant, le pantalon de correction de silhouette comprenant :
une bande de rotation de cuisse 106a enroulée en spirale dans une direction de rotation extérieure de la cuisse de telle sorte qu'elle s'étende vers le bas du bas-ventre à l'extrémité supérieure de la patella ; et
une bande de support de muscle grand glutéal 102 conçue pour supporter la partie d'extrémité inférieure du muscle grand glutéal de telle sorte qu'elle s'étende vers le bas et vers l'arrière du bas-ventre aux côtés gauche et droit et qu'elle soit reliée l'une à l'autre sur le côté inférieur de la hanche,
la bande de rotation de cuisse 106a étant tissée de façon à présenter une densité relativement plus élevée que d'autres parties du pantalon et une bande de support de muscle grand glutéal arrière 102b positionnée sur le côté inférieur de la hanche dans la bande de support de muscle grand glutéal 102 étant tissée de façon à présenter une densité relativement plus élevée qu'une bande de support de muscle grand glutéal avant 102a positionnée sur le bas-ventre dans la bande de support de muscle grand glutéal 102.

3. Pantalon de correction de silhouette selon la revendication 2, comprenant en outre une bande de rotation de muscle moyen glutéal 108 conçue pour entourer le muscle moyen glutéal de telle sorte qu'elle s'étende vers l'avant du côté supérieur de la hanche aux côtés gauche et droit et qu'elle soit reliée, au niveau des extrémités, à un côté de la bande de rotation de cuisse avant 106a, une bande de rotation de muscle moyen glutéal arrière 108b positionnée sur le côté supérieur de la hanche dans la bande de rotation de muscle moyen glutéal 108 étant tissée de façon à présenter une densité relativement plus élevée qu'une bande de rotation de muscle moyen glutéal avant 108a positionnée sur le côté avant d'un corps humain dans la bande de rotation de muscle moyen glutéal 108.

4. Pantalon de correction de silhouette selon la revendication 1, comprenant en outre une bande de support de genou 110 conçue pour entourer la région adjacente à la patella selon une forme sélectionnée parmi un losange, un hexagone et un cercle, la bande de support de genou 110 étant tissée de façon à présenter une densité relativement plus élevée que la bande de rotation de cuisse 106a et à présenter une densité relativement plus faible que la bande de rotation de mollet 106c.

5. Pantalon de correction de silhouette selon la revendication 4, comprenant en outre une bande de support de genou arrière 106b enroulée en spirale dans une direction de rotation extérieure à partir de l'extrémité supérieure de la bande de support de genou 110 de telle sorte qu'elle soit reliée à l'extrémité inférieure de la bande de support de genou 110, la bande de support de genou arrière 106b étant tissée de façon à présenter la même densité que la bande de support de genou 110.
